(19) Europäisches Patentamt
European Patent Office
Office européen des brevets

(11) Publication number: **0 373 919 B1**

(12) # EUROPEAN PATENT SPECIFICATION

(45) Date of publication of patent specification: **31.05.95**

(51) Int. Cl.6: **C07H 19/173**

(21) Application number: **89313047.6**

(22) Date of filing: **13.12.89**

(54) **Process for the preparation of 2'-deoxy-beta-adenosine.**

(30) Priority: **13.12.88 JP 313017/88**

(43) Date of publication of application:
**20.06.90 Bulletin 90/25**

(45) Publication of the grant of the patent:
**31.05.95 Bulletin 95/22**

(84) Designated Contracting States:
**DE FR GB**

(56) References cited:
**EP-A- 0 173 059**
**EP-A- 0 175 004**

**CHEMICAL ABSTRACTS, vol. 86, no. 15, 11th April 1977, page 547, abstract no.106950a, Columbus, Ohio, US; T. ITOH et al.: "Synthetic studies of potentialantimetabolites. XXI. Product distribution in the ribosylation reactions ofadenine and 1-deazapurine in the presence of stannic chloride"**

(73) Proprietor: **YUKI GOSEI KOGYO CO., LTD.**
**3-24, Hirakawa-cho 2-chome**
**Chiyoda-ku**
**Tokyo 102 (JP)**

Proprietor: **Japan Tobacco Inc.**
**2-1 Toranomon, 2-Chome**
**Minato-Ku**
**Tokyo 105 (JP)**

(72) Inventor: **Yoshikoshi, Hajime Tokyo Laboratoy**
**Yuki Gosei Kogyo Co. Ltd.**
**37-1 Sakashita 3-chome**
**Itabashi-ku**
**Tokyo 174 (JP)**
Inventor: **Itoh, Kazuo Tokyo Laboratoy**
**Yuki Gosei Kogyo Co. Ltd.**
**37-1 Sakashita 3-chome**
**Itabashi-ku**
**Tokyo 174 (JP)**
Inventor: **Naoi, Yoshitake Tokyo Laboratoy**
**Yuki Gosei Kogyo Co. Ltd.**
**37-1 Sakashita 3-chome**
**Itabashi-ku**
**Tokyo 174 (JP)**

**EP 0 373 919 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

J. AM. CHEM. SOC., vol. 106, 1984, pages 6379-6382, American Chemical Society;Z. KAZIMIERCZUK et al.: "Synthesis of 2'-deoxytubercidin, 2'-deoxyadenosine,and related 2'-deoxynucleosides via a novel direct stereospecific sodium saltglycosylation procedure"

J. AM. CHEM. SOC., vol. 105, 1983, pages 4059-4065, American Chemical Society;M.J. ROBINS et al.: "Nucleic acid related compounds. 42. A general procedurefor the efficient deoxygenation of secondary alcohols. Regiospecific andstereoselective conversion of ribonucleosides to 2'-deoxynucleosides"

Inventor: **Kuwakami, Hiroshi Life Science Research Lab.**
**Japan tobacco Inc.**
**6-2 Umegaoka**
**Midori ku**
**Yokohama-shi**
**Kanagawa 227 (JP)**
Inventor: **Matsushita, Hajume Life Science Research Lab.**
**Japan tobacco Inc.**
**6-2 Umegaoka**
**Midori ku**
**Yokohama-shi**
**Kanagawa 227 (JP)**

(74) Representative: **Holmes, Michael John et al**
**Frank B. Dehn & Co.**
**Imperial House**
**15-19 Kingsway**
**London WC2B 6UZ (GB)**

## Description

The present invention relates to a process for the preparation of 2′-deoxy-β-adenosine of formula (I):

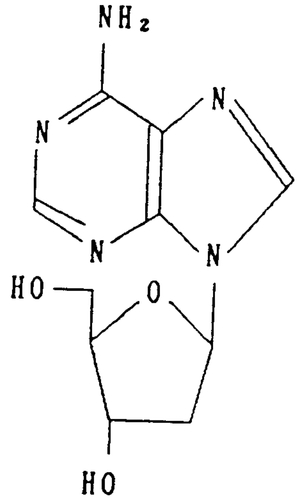

(I)

2′-Deoxy-β-adenosine is useful medicinally as an antiviral drug and as an antitumour agent. Further, 2′-deoxy-β-adenosine is a useful raw material for the preparation of 2′,3′-dideoxyadenosine which is well known as an anti-AIDS drug.

Conventionally, 2′-dexoy-β-adenosine may be prepared from natural deoxyribonucleic acid (DNA) by enzymatic degradation. However, such a process is not industrially applicable, because the availability of this raw material is restricted.

The following chemical synthetic methods for the preparation of 2′-deoxy-β-adenosine are known:

(i) Derivatives of adenine are condensed with derivatives of 2-deoxy-D-ribose to obtain an equivalent mixture of anomers with the subsequent selection of the β-anomer. (Biochimica et Biophysica Acta, 145, 221 (1967)).

(ii) The 2'-hydroxyl group of adenosine is eliminated to obtain 2'-deoxy-β-adenosine. (Journal of the American Chemical Society, 105, 4059 (1983); Journal of Organic Chemistry, 47, 485 (1982)).

However, neither of these known procedures for the production of 2'-deoxy-β-adenosine are suitable for industrial use. In (i), the selection and separation of the β-anomer from the mixture of anomers is too complicated. In (ii) the elimination of the hydroxyl group involves the use of poisonous tin compounds.

Recently, a synthesis of 2'-deoxy-β-adenosine has been reported where a sodium salt of 6-chloropurine derivative and 1-chloro-2-deoxy-3,5-di-O-p-toluoyl-α-D-erythro-pentafuranose are subjected to β-selective condensation. (Journal of the American Chemical Society, 106, 6379 (1984); TOKKYO-KOKAI-KOHO (18-month Publication of Unexamined Patent Application) SHOWA 61(1986)-106594).

This process has the disadvantages that:

(1) The 6-chloropurine derivative used as a raw material is expensive;

(2) sodium hydride, which is used in the preparation of a sodium salt, is hazardous; and

(3) Heating and pressurization are required for the substitution of a chloro group with an amino group.

Consequently, conventional processes are not industrially practicable.

Heterocycles 1976, 5(1), pp 285-292 describes the ribosylation reactions of adenine and 1-deazapurine with

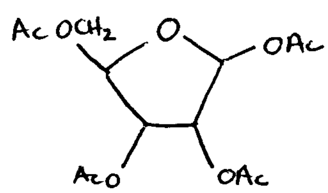

in the presence of stannic chloride.

The object of the present invention is to provide an industrial process for the preparation of 2'-deoxy-β-adenosine, that provides a high yield of 2'-deoxy-β-adenosine and a high selectivity of the β-anomer, without the use of expensive or hazardous raw materials.

The inventors investigated a process for the selective and efficient preparation of 2'-deoxy-β-adenosine of formula (I). They found that Compound (II) hereinafter can be obtained with a selectivity of at least 90% of β-anomer by the condensation of Compound (V) with Compound (IV) hereinafter in the presence of amines of the general formula (III) hereinafter using 1,2,4-trichlorobenzene as a solvent. Compound (I) can then be obtained by the elimination of the protecting groups from Compound (II).

Thus, the present invention relates to a process for the preparation of 2′-deoxy-β-adenosine characterized in that a derivative of 1-α-halogeno-2-deoxyribose with the general formula (V):

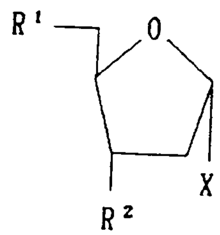

(V)

(wherein $R^1$ and $R^2$ are protected hydroxyl groups and X is a halogen atom)
is allowed to react with a derivative of adenine with the general formula (IV):

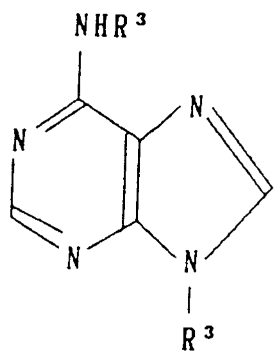

(IV)

(wherein $R^3$ is a triorganosilyl group)
in the presence of nitrogen bases of the general formula (III):

$(R^4)_nNH_{(3-n)}$ (III)

(wherein $R^4$ is an alkyl group or a triorganosilyl group, and n is an integer of zero to three)
in 1,2,4-trichlorobenzene, as a solvent to yield 3′,5′-disubstituted-2′-deoxy-β-adenosine of the following general formula (II):

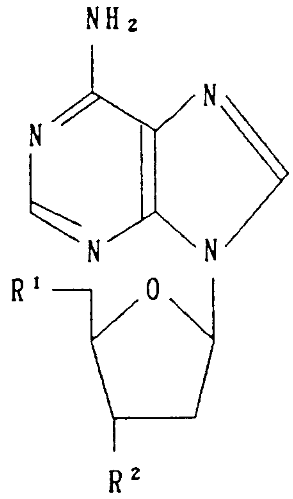

(II)

(wherein $R^1$ and $R^2$ are as defined above)

and then the protecting groups thereof are eliminated to yield 2′-deoxy-β-adenosine of the following formula (I):

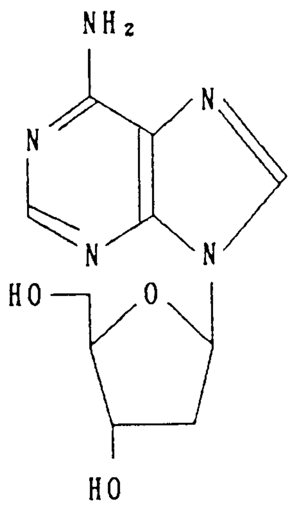

(I)

The synthetic method for the preparation of 1-α-halogeno-2-deoxyribose derivative (V) is described in J.J. Fox, N.C. Yung, I.Wempen, and M.Hoffer, J. Am. Chem. Soc., 83. 4066 (1961).

$R^1$ and $R^2$ of 1-α-halogeno-2-deoxyribose derivative (V), which is one of the raw materials used in the present invention, are protected hydroxyl groups, and the protecting groups used generally for saccharides may be used as the protecting groups thereof. Preferably, protecting groups, which can be easily eliminated under mild conditions are used. Such preferred protecting groups include aralkyl groups such as benzyl and trityl, acyl groups e.g. alkanoyl such as acetyl, propionyl, pivaloyl and benzoyl, alkyloxycarbonyl groups such as ethoxycarbonyl, aryloxycarbonyl groups such as phenoxycarbonyl, and the like. The protecting groups are not limited to the groups as mentioned above.

Where these protecting groups have a phenyl group, they may have as a substituent an alkyl group, a halogen atom, a nitro group, an alkoxyl group and the like.

X in the general formula (V) is a halogen atom, for example chlorine, bromine or iodine.

The synthetic method for the preparation of adenine derivatives of formulae (IV) is described in T.Nishimura and I.Iwai, Chem. Pharm. Bull., 12, 352 (1964).

$R^3$ in adenine derivatives of formula (IV) may, for example, be a triorganosilyl group such as trimethylsilyl, t-butyldimethylsilyl or phenyldimethylsilyl which are used as protecting groups for hydroxyl groups. However, $R^3$ is not limited to the groups mentioned above.

Condensation of Compound (V) with Compound (IV) is carried out in the present of nitrogen bases of formula (III).

The $R^4$ substituent of the nitrogen bases may, for example, be an alkyl group such as methyl, ethyl and butyl, or a triorganosilyl group such as trimethylsilyl. The nitrogen bases therefore include ammonia, triethylamine and hexamethyldisilazane. However, $R^4$ is not limited to the groups mentioned above.

1 equivalent of the Compound (V) is advantageously condensed with at least 1 equivalent, preferably 2 to 10 equivalents, of the Compound (IV) in the presence of 0.1 to 1.5 equivalents, preferably 0.5 to 1.5 equivalents of a nitrogen base of formula (III) in 1,2,4,-trichlorobenzene.

The condensation is carried out at a temperature of 0 °C to 100 °C, preferably 0 ° to 50 °C.

The condensation time depends on the reaction temperature, but generally terminates in a period of 0.5 to 20 hours.

After the condensation, Compound (II) may include small amounts of α-anomer and therefore be purified by silica-gel chromatography or recrystallization to obtain just the β-anomer; the protecting group may then be eliminated, for example, by reduction, hydrolysis or alcoholysis to obtain 2′-deoxy-β-adenosine (I). The yield based on Compound (V) may be 40% or more.

The present invention will be illustrated by the following, non-limiting example:

Example 1

1) Synthesis of 3′,5′-bis(4-chlorobenzoyloxy)-2′-deoxyadenosine

Air in a 500 ml three-necked flask (equipped with a reflux condenser, a thermometer and a stirrer) was replaced by nitrogen gas. 27.9 g (100 mmol) of $N^6$,$N^9$-bistrimethylsilyladenine (synthesized from adenine according to the method of T.Nishimura and I.Iwai, Chem. Pharm. Bull., 12, 352 (1964)), 4.5 g (45 mmol) of triethylamine and 200 ml of 1,2,4-trichlorobenzene were then placed in the flask forming a solution.

21.5 g (50 mmol) of 3,5-bis(4-chlorobenzoyloxy)-2-deoxy-α-D-ribofuranosyl chloride (synthesized from 2-deoxyribose according to the method of J.J.Fox, N.C.Yung, I.Wempen, and M.Hoffer, J. Am. Chem. Soc., 83, 4066 (1961)) were added to the solution with stirring and the condensation was carried out at room temperature for 10 hours.

Following the termination of the reaction, 100ml of water was added to the reaction mixture, separating the adenine. After the recovery of the adenine, the organic layer obtained was concentrated, producing 13.2 g of crude 3',5'-bis(4-chlorobenzoyloxy)-2′-deoxyadenosine.

This product was a mixture of both α-anomer and β-anomer in the ratio of approximately 10:90.

This crude product was purified by silica-gel column chromatography, using a solvent comprising a mixture of chloroform and methanol (volume ratio 100:5) to obtain 12.1 g (yield 46 %) of 3′,5′-bis-(4-chlorobenzoyloxy)-2′-deoxy-β-adenosine uncontaminated with the α-anomer.

Specific rotation : $[\alpha]_D^{20}$ = -4.54

$^1H$ NMR spectrum (500 MHz, $CDC1_3 + D_20$)

(ppm) 2.83, 3.44 (m, ddd, 2H, H-2′)

4.62 (m, 1H, H-4′)

4.68, 4.77 (dd, dd, 2H, H-5′)

5.84 (dt, 1H, H-3′)

6.51 (dd, 1H, H-1′)

7.44 - 8.01 (m,8H, aromatic ring proton)

8.32 (S, 1H, H-8)

2) Synthesis of 2′-deoxy-β-adenosine

12.1 g (23 mmol) of 3′,5′-bis(4-chlorobenzoyloxy)-2′-deoxy-β-adenosine were dissolved in 200 ml of methanol in a 500 ml three-necked flask equipped with a reflux condenser, a thermometer and a stirrer, and to the solution was added 20 ml of concentrated ammonia. The mixture was allowed to react at room temperature for 24 hours.

After the termination of the reaction, the reaction mixture was concentrated under reduced pressure, and the residue obtained was dissolved in water. After the solution was washed with chloroform, the aqueous layer was concentrated under reduced pressure. The crystals obtained were recrystallized from water to obtain 5.5 g (yield 89%) of 2′-deoxy-β-adenosine.

## Claims

**1.** A process for the preparation of 2′-deoxy-$\beta$-adenosine of formula (I):

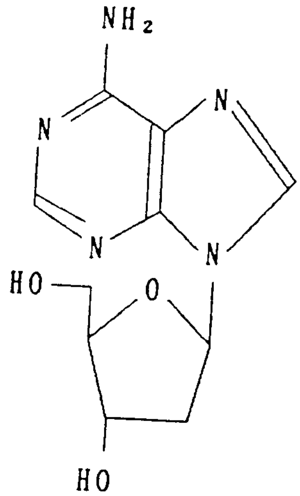

(I)

characterized in that a derivative of 1-$\alpha$-halogeno-2-deoxyribose of general formula (V):

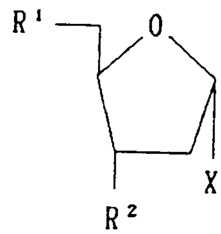

(V)

(wherein $R^1$ and $R^2$ are protected hydroxyl groups and X is a halogen atom)
is allowed to react with a derivative of adenine of general formula (IV):

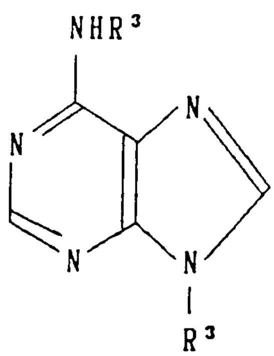

(IV)

(wherein $R^3$ is a triorganosilyl group)
in presence of a nitrogen base of general formula (III):

$(R^4)_nNH_{(3-n)}$ (III)

(wherein $R^4$ is an alkyl group or a triorganosilyl group, and n is an integer of zero to three)
in 1,2,4-trichlorobenzene as a solvent to yield 3′,5′-disubstituted-2′-deoxy-$\beta$-adenosine of following general formula (II):

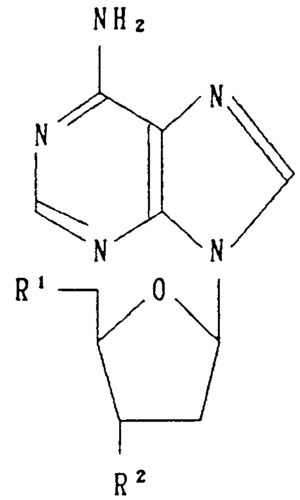

(II)

(wherein $R^1$ and $R^2$ are as defined above) and the protecting groups thereof are subsequently eliminated.

**2.** A process as claimed in claim 1, where $R^1$ and $R^2$ of 1-α-halogene-2-deoxyribose derivative (V) are aralkyl groups, acyl groups, alkyloxy carbonyl or arylcarbonyl groups.

**3.** A process as claimed in either of claims 1 and 2, wherein 1 equivalent of Compound (V) is condensed with 2-10 equivalents of Compound (IV).

**4.** A process as claimed in any of claims 1 to 3, wherein the condensation of compounds (IV) and (V) is carried out at a temperature of 0-50 °C.

**Patentansprüche**

**1.** Verfahren zur Herstellung von 2'-Deeoxy-β-adenosin der Formel (I):

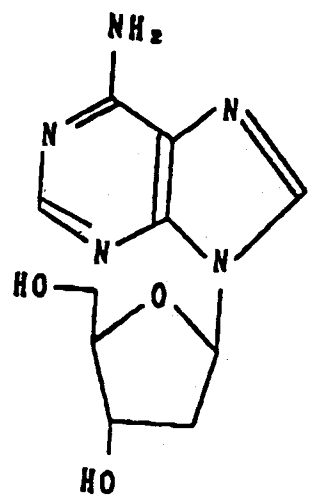

(I)

dadurch gekennzeichnet,
daß ein Derivat von 1-α-Halogen-2-desoxyribose der nachfolgenden Formel (V):

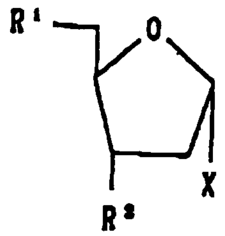

(V)

(wobei $R^1$ und $R^2$ geschützte Hydroxyl-Gruppen und X ein Halogenatom sind) umgesetzt wird mit einem Adeninderivat der allgemeinen Formel (IV):

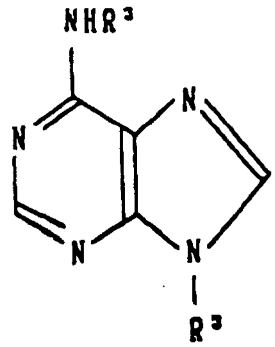

(IV)

(wobei $R^3$ eine Triorganosilyl-Gruppe ist) in Gegenwart einer Sticketoffbase der allgemeinen Formel (III):

$(R^4)_nNH_{(3-n)}$ (III)

(wobei $R^4$ eine Alkyl-Gruppe oder eine Triorganosilyl-Gruppe, und n ein Ganzzahliges von Null bis drei sind) in 1,2,4-Trichlorbenzol als Lösungsmittel, um 3',5'-disubstituiertes-2-Desoxy-$\beta$-adenosin der nachfolgenden allgemeinen Formel (II):

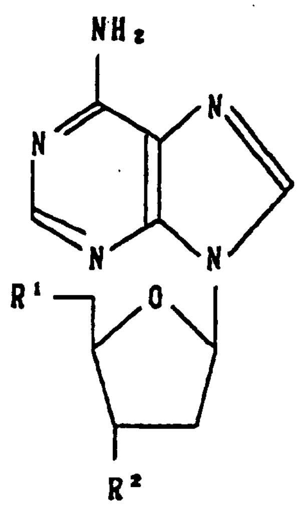

(II)

(wobei $R^1$ und $R^2$ die oben angegebenen Bedeutungen aufweisen) zu erhalten und die Schutzgruppen dieser Verbindung enschließend eliminiert werden.

**2.** Verfahren nach Anspruch 1, wobei $R^1$ und $R^2$ des 1-$\alpha$-Halogen-2-desoxyribose-Derivats (V) Aralkyl-Gruppen, Acyl-Gruppen, Alkyloxycarbonyl- oder Arylcarbonyl-Gruppen sind.

3. Verfahren nach einem der Ansprüche 1 und 2, wobei 1 Äquivalent der Verbindung (V) mit 2-10 Äquivalenten der Verbindung (IV) kondensiert wird.

4. Verfahren nach einem der Ansprüche 1 bis 3, wobei die Kondensation der Verbindungen (IV) und (V) bei einer Temperatur von 0-50 °C durchgeführt wird.

**Revendications**

1. Procédé pour la fabrication de 2'-déoxy-$\beta$-adénosine de formule (I) :

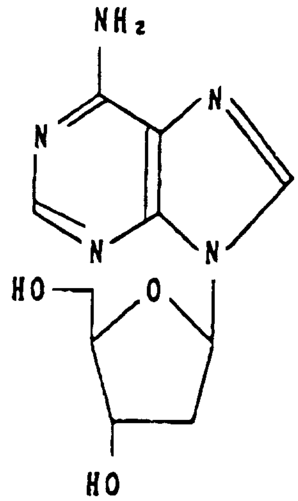

(I)

caractérisé en ce que l'on fait réagir un dérivé de 1-$\alpha$-halogéno-2-déoxyribose de formule générale (V) :

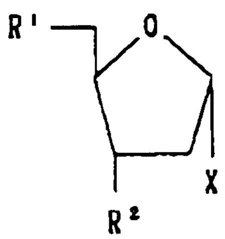

(V)

(dans laquelle $R^1$ et $R^2$ sont des groupes hydroxyles protégés et X est un atome d'halogène) avec un dérivé d'adénine de formule générale (IV) :

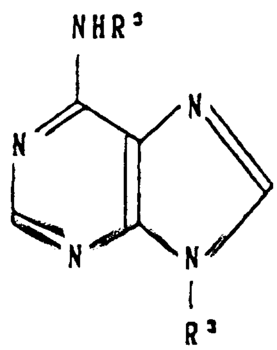

(IV)

(dans laquelle $R^3$ est un groupe triorganosilyle) en présence d'une base azotée de formule générale (III) :

$(R^4)_nNH_{(3-n)}$ (III)

(dans laquelle $R^4$ est un groupe alkyle ou un groupe triorganosilyle et n est un entier de 0 à 3) dans le 1,2,4-trichlorobenzène comme solvat pour donner une 2'-déoxy-$\beta$-adénosine 3',5'-disubstituée de formule générale (II) suivante :

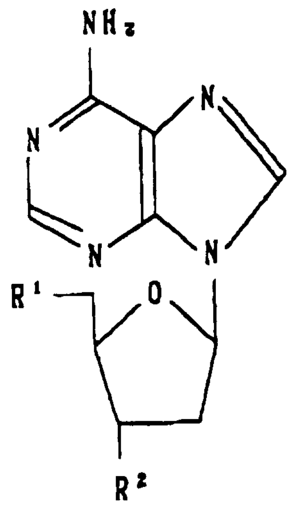

(II)

(dans laquelle $R^1$ et $R^2$ sont définis comme ci-dessus) et ensuite on élimine ses groupes protecteurs.

2. Procédé selon la revendication 1, dans lequel $R^1$ et $R^2$ dans le dérivé de 1-$\alpha$-halogéno-2-déoxyribose (V) sont des groupes aralkyles, des groupes acyles, des groupes alkyloxycarbonyles ou des groupes aryloxycarbonyles.

3. Procédé selon la revendication 1 ou 2, dans lequel on condense 1 équivalent du composé (V) avec 2-10 équivalents du composé (IV).

4. Procédé selon l'une quelconque des revendications 1 à 3, dans lequel la condensation des composés (IV) et (V) est effectuée à une temperature de 0-50 °C.